# EUROPEAN PATENT SPECIFICATION

(11) **EP 4 044 789 B1**
(45) Date of publication and mention of the grant of the patent: **21.01.2026**
(21) Application number: 20788893.4
(22) Date of filing: 28.09.2020
(51) Int. Cl.: A01C 21/00, G01N 1/14

(54) **SOIL WATER COLLECTION AND ANALYSIS SYSTEM**
SYSTEM ZUR SAMMLUNG UND ANALYSE VON BODENWASSER
SYSTÈME DE COLLECTE ET D'ANALYSE D'EAU DU SOL

(30) Priority: 16.10.2019 US 201962916180 P; 12.11.2019 US 201962934049 P
(43) Date of publication of application: 24.08.2022
(73) Proprietor: Precision Planting LLC, Tremont, IL 61568 (US)
(72) Inventor: KOCH, Dale, Tremont, IL 61568 (US); MINARICH, Nicholas, Tremont, IL 61568 (US); SWANSON, Todd, Tremont, IL 61568 (US); NELSON, Rachel, Tremont, IL 61568 (US); VACCARI, Adam, Tremont, IL 61568 (US)
(74) Representative: AGCO Intellectual Property Department
(86) International application number: PCT/IB2020/059043
(87) International publication number: WO 2021/074722

(56) References cited:
- CN-A- 109 884 276
- CN-U- 206 387 608
- DE-A1- 102017 106 121
- DE-A1- 19 617 106
- US-A- 5 246 862
- US-A- 5 465 628
- US-A1- 2014 165 713
- US-A1- 2018 112 430

## Description

### BACKGROUND

The invention relates generally to a systems for collecting and analyzing soil water samples.

Soil analysis and testing is an important aspect of the agricultural arts. Test results provide valuable information on the chemical makeup and characteristics of the agricultural soil (e.g. levels of nitrogen, phosphorous, potassium, etc.) so that various amendments may be added to the soil to maximize the quality and quantity of crop production. Samples are generally processed to quantify the presence of particular analytes (i.e. substances or chemical constituent of interest), which can then be used to ascertain the amounts and types of soil amendments and fertilizers required for various areas of the agricultural field.

Improvements in soil testing and analysis are desired.
US 2014/0165713 A1 discloses a system for nutrient monitoring in an agricultural field to provide real time analysis and monitoring of nutrients using ion selective electrodes, in which a sampling assembly includes a manifold and a plurality of sampling lines, with one or more micropumps coupled to the manifold and sampling lines.
CN206387608U discloses a soil sampling and remote monitoring device for sampling different soil solutions in a karst area, in which a vacuum pump is used to extract a soil solution at different depths.

### BRIEF SUMMARY

According to the invention there is provided a system for collecting and analyzing soil water samples, as defined by the appended claims.

The present disclosure provides a system for collecting and analyzing soil pore water samples to determine plant nutrient or other parameter levels/concentrations in the soil which may be relevant for agricultural purposes in one non-limiting application. The soil water collection and analysis system incorporates the soil pore water extraction/collection, processing, and chemical analysis functions into a single unified equipment platform which is portable and may be remotely deployed in the agricultural field. The soil water collection and analysis system operates by capturing or extracting a soil water sample, which will contain the particular analytes of interest (e.g. nitrogen, potassium, or other), combining the sample with one or more reagents specifically selected to cause a chemical reaction with the targeted analyte(s), and analyzing the resultant property change in the water-reagent mixture (e.g. color, pH, or other aspect/parameter) from the interaction between the reagent and analyte present in the collected sample which will be indicative of the level of analyte present. Any suitable analytical technique and device may be used to determine the level or amount of analyte present. Non-limiting examples include colorimetry, Ion Selective Electrodes (ISE's), and Ion exchange Resins. The system may be configured and operable to analyze any variety of analytes or parameters of the soil (e.g. levels of nitrogen, phosphorous, potassium, pH, temperature, etc.).

Some of the component parts of the system for collecting the water samples may be configured for semi-permanent or permanent stationary and direct implantation into the soil of the agricultural field, or alternatively may be a part of a mobile system mounted to a moving agricultural vehicle (e.g. towed, pushed, or self-propelled). For stationary implantation in soil, a lysimeter-type soil water sample collection probe (alternatively "sample probe" for short as also used herein) is provided. The sample probe may have an elongated generally tubular body comprising a filter media for direct contact with the soil and interior cavity for collecting the filtered water sample (filtrate). The probe may have a rounded, cone-shaped, or other readily implantable shaped end.

The sample probe is fluidly and operably coupled to a sample processing sub-system portion of the soil water collection and analysis system. The sample processing sub-system may be housed in an in situ equipment enclosure which contains the active devices, electronics, fluid components, and others needed for processing and chemically analyzing the collected filtrate from the sample probe for determining the levels of soil nutrients or other relevant parameters (e.g. pH, etc.). The equipment enclosure may be closely coupled to the probe in the field in close proximity, such as adjacent thereto and/or physically coupled to the probe at the soil sampling site. In such an implementation, the combination of sample probe and sample processing sub-system may therefore define and be configured to serve as a remote fully functional soil water analysis lab or station. The sampling station is operable to both collect and fully analyze the soil water sample from the agricultural field. Optionally, the sampling station may communicate the collected data and analysis results externally to a remote programmable processor-based central control system which acts as a repository for soil analysis data and storage. This soil water sampling station or unit may contain a probe controller comprising one or more programmable microprocessors. The controller may be configured via appropriate software or program instructions to control the entire operation of sampling station. The probe controller may include a communications interface which is communicably linked (e.g. wirelessly) to one or more remote electronic devices for data sharing and/or control signal interchange. Accordingly, the soil water collection and analysis system embodied in such a sampling station may be fully automated for collecting and analyzing the soil moisture sample to detect the presence and level of an analyte.

A sampling network comprising a plurality of sampling stations each including the combination of a soil water sample collection probe and a sample processing sub-system may be implanted in various spatially-separated locations throughout the agricultural field. Each sampling station may be communicably linked via the cloud (i.e. cloud computing) or other wireless communication means to the one or more remote electronic devices; at least one of which may serve as a processor-based central control system to exchange data and control signals with the sampling station. The central control system device which becomes a central or main controller may be configured to direct and control the operation of these remote sampling stations. Each sampling station may be configured to analyze the same or different analytes (e.g. nitrogen) which the central control system may use to generate both a moisture and chemical profile of various regions of the agricultural field indicating specific nutrient levels for each. This information allows the end user (e.g. farmer) to know which areas are deficient in various soil nutrients so that application of suitable types and amounts of soil amendments/fertilizers can be better planned in advance. The control system may be configured to simultaneously process and analyze data received from a plurality of soil water sampling stations in parallel in real-time, thereby saving analysis time and allowing the user to see a snapshot of all soil nutrient levels at once.

The sample probe may be a modular type sampling probe system comprising one or more extension members and one or more sampling modules each which include a filter media. The sampling modules and extension members may be detachably coupled together to form various configurations and lengths of the modular sampling probe for collecting soil water samples at a single predetermined soil depth, or at several different depths using the same modular unit. The modular system may include anti-rotational couplings at the joints between different sections and appurtenances of the modular sample probe. The anti-rotational couplings may comprise splined interfaces formed by complementary configured male and female splined couplings which provide a torque resistant jointed assembly for rotatably implanting the probe into the soil that overcomes resistance imparted to the probe by the soil. The modular probe sampling system advantageously allows the user or manufacturer to easily customize the water collection depth(s) in the soil using a plurality of interchangeable and detachably coupled components preferably sharing a common anti-rotational mounting interface.

Although the present soil water analysis system may be described herein with reference to quantification of plant nutrient and related parameters of the soil, the system is expressly not limited in its applicability to agricultural-related uses alone. Accordingly, the soil water analysis system may be readily adapted for use to quantify other type chemical characteristics (e.g. heavy metals, arsenic, lead, etc.) which are relevant to a variety of groundwater and soil pore water routine monitoring applications for soil contaminants in industries such as mining, petroleum, power generation plant waste basin monitoring, landfills, and others.

### BRIEF DESCRIPTION OF THE DRAWINGS

The present disclosure will become more fully understood from the detailed description and the accompanying drawings, wherein like elements are labeled similarly and in which:
FIG. 1 is a schematic diagram of a soil water sampling system comprising a water sampling probe and associated sample processing sub-system including a vacuum device for applying negative pressure to the probe;
FIG. 2 is a side view of the sampling probe embedded in soil for capturing pore water;
FIG. 3 is a side view showing a vacuum device for applying negative pressure to the probe;
FIG. 4 is schematic diagram of a cloud-based networked communication system for sharing soil water analysis data and interchanging command signals to operate portions of the sampling system;
FIG. 5 is a side view of a first method for embedding the sample probe in the soil and establishing intimate contact between the probe and soil for extracting a water sample;
FIG. 6 is a side view of a second method for embedding the sample probe in the soil and establishing intimate contact between the probe and soil for extracting a water sample;
FIG. 7 is a side view of a third method for embedding the sample probe in the soil and establishing intimate contact between the probe and soil for extracting a water sample;
FIG. 8 is a side view of a fourth method for embedding the sample probe in the soil and establishing intimate contact between the probe and soil for extracting a water sample;
FIG. 9 is a side view of a sample probe with external threads;
FIG. 10 is a first side view of a hydrophilic type sampling probe in the form of a testing cassette for a system according to the invention;
FIG. 11 is a perspective view thereof;
FIG. 12 is a second side cross sectional view thereof;
FIG. 13 is a side view of a portion of the reel of cassette tape with test pad thereon of the sampling probe of FIG. 10;
FIG. 14 is a side view of a multi-layer composite block or test pad on the cassette tape of the sampling probe of FIG. 10;
FIG. 15 shows a mechanical method for liberating captured water for a saturated test pad of the sampling probe of FIG. 10;
FIG. 16 shows a chemical method for liberating captured water for a saturated test pad of the sampling probe of FIG. 10;
FIG. 17 shows an alternative powered sampling tape drive of testing cassette of FIG. 10 configured for use with a sampling tape which includes a removably protective overlay film;

All drawings are schematic and not necessarily to scale. Components numbered and appearing in one figure but appearing un-numbered in other figures are the same unless expressly noted otherwise. A reference herein to a whole figure number which appears in multiple figures bearing the same whole number but with different alphabetical suffixes shall be constructed as a general refer to all of those figures unless expressly noted otherwise.

### DETAILED DESCRIPTION

The features and benefits of the invention are illustrated and described herein by reference to exemplary ("example") embodiments. This description of exemplary embodiments is intended to be read in connection with the accompanying drawings, which are to be considered part of the entire written description. Accordingly, the disclosure expressly should not be limited to such exemplary embodiments illustrating some possible non-limiting combination of features that may exist alone or in other combinations of features.

In the description of embodiments disclosed herein, any reference to direction or orientation is merely intended for convenience of description and is not intended in any way to limit the scope of the present invention. Relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise.

As used throughout, any ranges disclosed herein are used as shorthand for describing each and every value that is within the range. Any value within the range can be selected as the terminus of the range. In the event of a conflict in a definition in the present disclosure and that of a cited reference, the present disclosure controls.

### Vacuum Type Sampling System

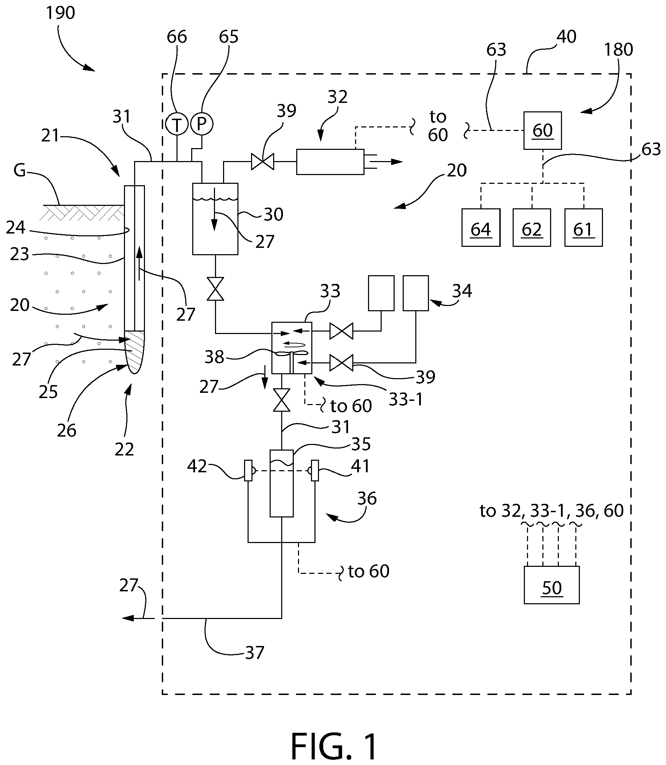

FIG. 1 is a schematic flow diagram of a soil water analysis system comprising a soil water sample collection lysimeter or probe 20 and an associated closely coupled sample processing sub-system 180. It bears noting that the sample processing sub-system 180 is mounted in the agriculture field proximate and preferably adjacent to the probe 20 which captures the water sample. This combination advantageously defines and provides a complete soil water analysis sampling station 190 with capabilities to extract, process, and analyze the water sample in situ and in real-time to quantify an analyte of interest (e.g. soil nutrients). The system and its components are further described in turn below.

Sample probe 20 may have an elongated tubular housing or body including a probe longitudinal axis LA, a top end 21, bottom end 22, and circumferentially-extending sidewall 23 extending axially between the ends. The sidewall may have an at least partially solid structure for at least part of its length, or a majority of its length in some constructions. The top end 21 may be closed and sealed except for passage of a flow conduit therethrough as further described herein. The body may be formed of any suitable metallic or non-metallic material including polymeric materials (e.g. PVC, polyethylene, etc.) and preferably corrosion resistance metals if used (e.g. aluminum or stainless steel). The probe body is configured for implantation in the soil S beneath the ground surface G.

An internal cavity 24 extends axially between the top and bottom ends 21, 22 of the probe for collection water from the adjacent soil. The probe sidewall 23 which defines the cavity may be cylindrical forming a circular transverse cross-sectional shape. Sidewall may have other non-polygonal or polygonal cross-sectional shapes (e.g. square, rectangular, hexagonal, octagonal, etc.). The bottom end 22 of probe 20 may have any suitable shape. Bottom end 20 may be simply flat or straight (i.e. perpendicular to probe longitudinal axis LA) as shown in FIG. 24. Bottom end 22 may alternatively be shaped to facilitate insertion into the soil, such as for example rounded as shown, conical or spike shaped, or otherwise.

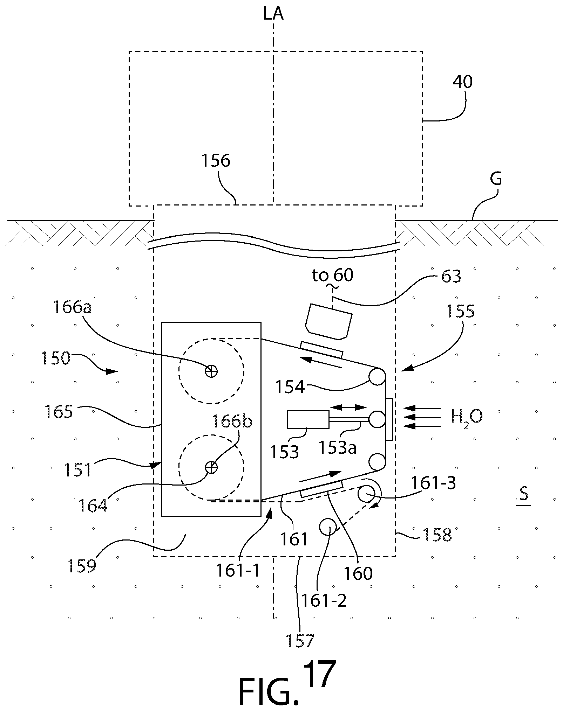

Probe 20 further includes a porous filter or media 26 configured for drawing water from the soil into the interior of the probe for collection and analysis. The porous media may comprise a porous tip 25 mounted to the bottom end 22 of the probe. The tip 25 may extend upwards from the bottom end for a distance along the sides of the probe as shown. As shown in FIGS. 17-30, the porous media 26 in the form of a porous filter 220 may form partial or complete circumferential cylindrical segments forming a portion of the sidewall 25 of probe 20, as further described herein. The bottom end 22 of probe 20 may have a solid non-porous flat, conical, or other shaped structure.

The porous media may be formed of any suitable material having a suitable pore or opening size or selected to preclude drawing particles of soil into the probe, but allow water to flow through the media. Suitable porous filter or media includes for example without limitation porous ceramic, sintered metal, polymers, polymeric or fibrous membranes, screens, etc.). The porous media may comprise affixing the porous media to an open section or multiple open sections of the sidewall 23 of the probe between the top and bottom ends 21, 22 to draw water laterally into the probe.

The soil water sample processing sub-system 180 associated with sample probe 20 further includes collection chamber 30 which receives the collected soil water sample, mixer 33-1 which comprises a mixing chamber 33, one or more reagent chambers 34, sample analysis chamber 35, vacuum pump 32, and a soil water sample analysis device 36 for measuring the concentration/level of analyte in the water sample. The porous media 26, collection chamber 30, mixing chamber 33, analysis chamber 35, and reagent chamber(s) 34 may be fluidly coupled together via suitable flow conduits 31 such as tubing (not all tubing has been numbered in FIG. 1 for clarity of depiction). Any suitable rigid or flexible non-metallic or metallic tubing material may be used such as polyethylene, PTFE (polytetrafluoroethylene), etc. Collection chamber 30 is fluidly coupled to the porous media 26 and mixing chamber 33. Each reagent chamber is fluidly coupled to the mixing chamber. The analysis chamber 35 which is in turn fluidly coupled to the mixing chamber. One or more valves 39 may be used to control and time the flow of water and reagents to the mixing chamber 38.

Mixing chamber 38 includes an electric motor driven mixing blade assembly 38. The blade assembly operates to mix the soil water sample and reagents to induce a chemical reaction which can be detected and the analyte concentration thus measured by the sample analysis device. The "mixing chamber" could also be any apparatus or method of mixing or moving solution around, such as a magnetic stir bar, circulation pump, shaker, or just plain perturbed.

Vacuum pump 32 may be electric motor driven and is fluidly coupled to the mixing chamber 38 via a flow conduit 31. Pump 32 is configured and operable to draw a vacuum through the porous media 26 for pulling water from the surrounding soil into the probe. The pump then draws the water sample into the mixing chamber from the media via a flow conduit 31 extending through the probe body, as shown. Any suitable commercially-available vacuum pump may be used for this application.

A suitable electric power source 50 may be provided and electrically coupled to the vacuum pump 32, mixing chamber 38, sample analysis device 36, and other components requiring power associated with probe 20. Power source 50 may be a rechargeable battery unit for remote placement of the sample probes in the agricultural field. A rechargeable solar batter unit including solar panel or sensor for recharging the batteries via sunlight may be used. This may be particularly advantageous considering the remote placement of the probe or probes in the field.

The sample analysis device 36 may be an electronic device comprising an absorbance measurement colorimeter which operates at a specific wavelength to detect the particular analyte of interest, such as for example without limitation about 525 nm for detection of a nitrate indicator or 210 nm for detection of nitrate itself, or others. It will be appreciated that in this example, colorimetric analysis and detection of nitrate at 525nm or 210 nm is only for a specific nitrate reduction reaction. Other analytes may require device 36 to operate at other wavelengths specific to detection of such other analytes. Sample analysis device 36 operably cooperates with analysis chamber 35 which may be a transparent vessels placed between the LED (light emitting diode) transmitting diode 41 and LED receiving diode 42. As the reacted water and reagent mixture flows past the device, the absorbance of particular wavelengths of light by the mixture is measured to quantify the concentration of the analyte in the water sample. Such colorimeters are commercially-available.

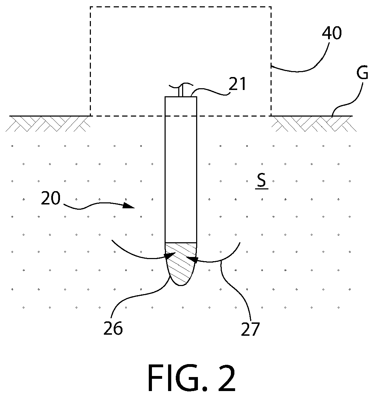

The collection chamber 30, mixing chamber 33, one or more reagent chambers 34, sample analysis chamber 35, vacuum pump 32, a soil water sample analysis device 36, power source 50, and other related appurtenances including valving and tubing may be mounted in a separate equipment enclosure 40 (schematically designated by the dashed box in FIG. 1) from the probe 20. The enclosure may have any suitable configuration for the probe installation site. In various constructions, the equipment enclosure may be mounted directly above or on top of the probe (see, e.g. FIG. 2), or in another other position adjacent to the probe. The top end 21 of the probe 20 may be positioned inside the enclosure as also shown in FIG. 2.

A method or process for collecting and analyzing a soil water sample using the system of FIG. 1 will now be briefly described. First, the sample probe 20 is implanted into the soil to be tested such that the porous media 26 is in intimate contact with the surrounding soil at the desired depth for collecting the water sample. A vacuum is then drawn by operating the vacuum pump 32. The water sample (see directional water flow arrows 27) is drawn transversely/horizontal through the porous media 26 into probe cavity 24, and then vertically upward from the bottom to top of the probe 24 parallel to the longitudinal axis LA. In other possible filter media arrangements, water could be drawn through the media in other directions or orientations (e.g. vertically or obliquely to axis LA). The collected water is then deposited into mixing chamber 30 via flow conduit 31. Operation of vacuum pump 32 is then stopped.

The extracted soil water sample next flows downward via gravity to mixing chamber 38 from collection chamber 30 by opening previously closed mixing chamber inlet valve 39. Collection chamber 30 may be omitted and the extracted soil water sample may instead be deposited directly into mixing chamber 38. Next, the reagent chambers 34 are then fluidly connected to the mixing chamber by opening the reagent valves 39 to introduce reagent into the sample. The mixing chamber inlet valve and reagent valves are then closed. The mixer is operated so that blade assembly 38 thoroughly mixes the reagent and water sample. This causes a chemical reaction which changes the color of the resulting water-reagent mixture. The previously closed mixing chamber outlet valve 39 is then opened which causes the water-reagent mixture to flow through the clear analysis chamber and outwards from equipment enclosure 40 through drain line 37. The mixing chamber inlet valve may optionally be opened if needed to break any vacuum created in the mixing chamber 33. As the water sample flows through the colorimeter (sample analysis device 36), the analyte (e.g. nitrate or other) is measured to determine its concentration in the sample which is indicative of the concentration or level of the analyte in the surrounding soil.

It bears noting that the volume of reagent held in the reagent chambers 34 may be more than necessary to process a single soil water sample. Accordingly, a single reagent charge or fill of the reagent chambers may be capable of processing multiple samples. In such an implementation, the reagent valves may be operated to dose the water sample in the mixing chamber 33 with only the amount necessary each time to cause a chemical reaction with the analyte for analysis.

The foregoing sampling and analysis process may be fully automated by providing a "smart" probe comprising a probe control system comprising a programmable probe controller 60 operably and communicably coupled to the foregoing components of the sample processing sub-system 180 via wireless and/or wired communication links 63 to control their operation and operating sequence in the manner described above. Controller 60 thus provides a user-configurable control system for the probe sampling system. Individual communication links to each component (e.g. valves, pump, chambers, mixer, sample analysis device, etc. which are not shown in FIG. 1 to avoid undue clutter). The controller 60 based control system may also be housed inside the equipment enclosure 40 which may be at least partially sealed from the weather and environment to protect the electronics.

The programmable probe controller 60 includes a programmable processor which may be one or more processors or microprocessors, a system on a chip (integrated circuit), one or more microcontrollers, ASICs (application specific integrated circuits), or combinations thereof. Probe controller 60 includes processing logic for executing programmable control logic or software instructions comprising one or more programs which are executed by the controller to control operation of the sampling system.

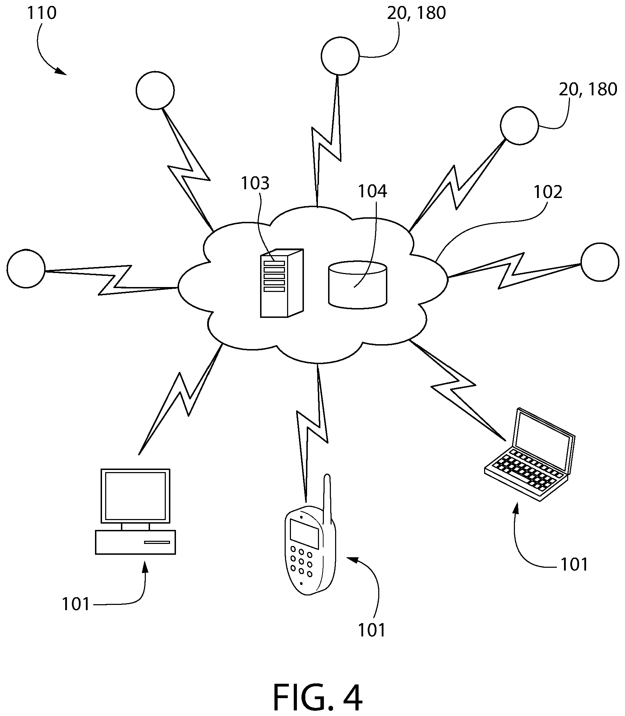

Probe controller may further include an input/output communication interface or module 61 configured for wireless and/or wired communication for programming the processor and exchanging sampling results or other data via external processor-based personal electronic devices (e.g. computer, cell phone, tablet, laptop, etc.). Communication module 61 therefore may be configured for transmitting and receiving communications and data via wireless and/or wired protocols to and from one or more user's personal electronic devices. This allows the user or users to be operably coupled to the probe controller 60 for uploading software to configure and control operation of the probe controller 60, and for downloading soil water sampling related measurement, analysis, and other data or results. The user's personal electronic device 101 may therefore act as processor-based main control system by running appropriately configured software, as further described elsewhere herein. The personal electronic devices 101 may be communicably coupled or linked to probe controller 60 either directly (e.g. Wi-Fi, near field communication (NRC), Bluetooth^{®}, coupled wired connection or jack, etc.), or alternatively via the Internet such as by cloud computing in which both the personal electronic device and probe controller 60 are in turn communicably linked via the "cloud" 102, as further described herein (see, e.g. FIG. 4).

Probe controller 60 further includes non-transitory tangible computer readable medium 62 operably coupled and accessible to the controller 60. The computer or machine accessible and readable medium may include any suitable volatile memory and non-volatile memory or devices operably and communicably coupled to the controller processor(s). Any combination and types of volatile or non-volatile memory may be used including as examples, without limitation, random access memory (RAM) and various types thereof (e.g. ferroelectric RAM, DRAM, etc.), read-only memory (ROM) and various types thereof, hard disk drives (HDD), solid-state drives (SSD), flash memory, SD card, USB drive, or other suitable memory and devices which may be written to and/or read by the processor operably connected to the medium. The non-volatile memory may thus be any permanent or removable type memory. Both the volatile memory and the non-volatile memory are used for saving data or results from processed samples, for storing programming (program instructions or software), and storing operating parameters associated with operation of the rotary machine 101 or processing samples, etc. Both the volatile memory and the non-volatile memory may be used for storing the program instructions or software.

The computer or machine accessible and readable non-transitory medium 62 (e.g. memory) contains executable computer program or software instructions which when executed by the probe controller 60 cause the system to perform operations or methods including measuring properties and testing of soil water sample. While the machine accessible and readable non-transitory medium 62 (e.g., memory) is shown to be a single medium, the term should be taken to include a single medium or multiple media (e.g., a centralized or distributed database, and/or associated caches and servers) that store the one or more sets of control logic or instructions. It is well within the ambit of one skilled in the art to provide and configure a controller with all the required appurtenances to provide a fully functional control system for operating the probe sampling system and processing soil water samples in the manner disclosed herein.

The probe controller 60 may further include a GPS module 64 which allows the controller to know its precise geo-coordinates where implanted in the agriculture field and transmit its location to the central control system 100 embodied by the user's personal electronic device. The control system 100 may thus act as a hub which keeps track of the locations of a plurality of probes 20 deployed in the agriculture field, as further described herein.

It bears noting that the probe control system includes all other usual appurtenances and ancillary devices known in the art necessary to form a fully functional and programmable probe control system.

One or more temperatures sensors 66 and pressure sensors 65 may be provided which are communicably coupled to the programmable probe controller 60. The temperature sensor 66 is arranged to measure a real-time actual temperature of the collected soil sample and transmit the measured temperature to the controller. The temperature sensor 66 may be located at any suitable point in the system to measure the water temperature. In one non-limiting example, the temperature sensor 66 may be arranged to measure the temperature of the collected water in the inlet flow conduit 31 to the collection chamber 30. Any suitable type commercially-available temperature sensor may be used (e.g. thermistor, RTD, etc.).

The pressure sensor(s) 65 is arranged to measure pressure of the collected water sample extracted from the soil via probe 20 under negative pressure (vacuum) created by the vacuum pump 32. Pressure sensor 65 is preferably located either in the probe or inlet flow conduit 31 upstream of the collection chamber 30. Any suitable commercially-available pressure sensor may be used.

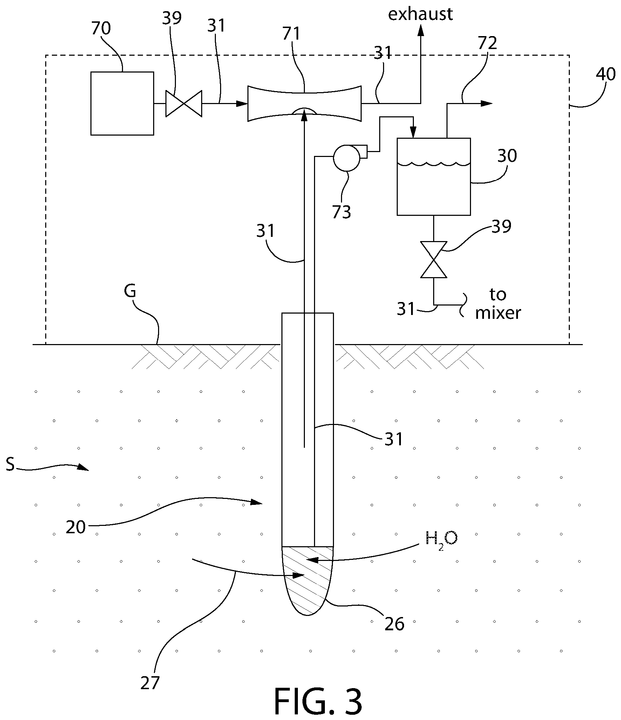

FIG. 3 shows an energy conservation version of the basic system of FIG. 1 which prolongs onboard battery life of the probe system. In this energy conserving system, the vacuum pump is replaced by a combination of an added small water pump 73 with low power consumption requirements and an air-powered venturi jet pump 71. The jet pump is only run for short time sufficient to create an initial negative pressure or vacuum inside the probe to start the inflow of water through the porous media 26 at the start of a sampling cycle. Operation of the jet pump is then terminated. A high pressure air source 70, which may be a canister or tank of pressurized air, is fluidly coupled to an air inlet port of the venturi jet pump 71 via an inlet flow conduit 31. The air tank may be mounted on, within, or adjacent to the equipment enclosure 40. A vacuum flow conduit 31 is fluidly coupled to the internal cavity 24 of probe 26 media 26 and an inlet to the jet pump 71 to draw a vacuum on the porous media. A separate water inlet flow conduit 31 is fluidly coupled to the porous media 26 and suction or inlet of the water pump 73.

In operation, the air discharge valve 39 fluidly coupled to the air tank is opened which establishes a flow of high pressure air through the jet pump 71. The motive force of the high pressure air flowing through the narrowed throat section of the jet pump with venturi shape draws a vacuum on the probe internal cavity 24, which induces an inflow of water from the surrounding soil inwards through the porous medium 26 into the probe cavity. Air discharge valve 39 is then closed. Next, water pump 73 may be started to draw and pump the extracted water from probe 20 into collection chamber 30. The pump may then be stopped and water is transferred via gravity to the mixing chamber 33 of mixer 33-1 for addition of reagent, mixing, and further processing through the system of FIG. 1 for analysis. The remainder of the sample analysis system and related analysis process is the same as previously described herein with respect to FIG. 1. Any suitable commercially-available venture jet pump may be used for this application.

The water pump 73 may be omitted and the high pressure air source 70 may be used instead to pressurize the probe cavity 24 and force collected water in the probe 20 through the water extraction flow conduit 31 into the collection chamber 30. In such a system, the high pressure air source 70 serves a dual function of both establishing an initial vacuum in the probe 20 to induce the inward flow of soil water and thereafter force the collected water upwards and into the collection chamber 30.

FIG. 4 is a system diagram of a networked date communication system 110 for establishing two-way wireless communications between one or more remote personal electronics devices and an array of sampling stations 190 using cloud computing previously mentioned above. Each sampling station 190 comprises at least one probe 20 and a sample processing sub-system 180). The "cloud" 102 acts as a communication hub which communicably and operably couples or links each personal electronic device 101 to the probe controller 60 in each of the probes 20 (see also FIG. 1 showing the probe controllers). The cloud in this situation hosts and runs appropriately configured soil analysis programs and other software (e.g. applications) configured to communicate and exchange information, operating instructions/programming, and data between each probe 20 deployed in the agriculture field and the user's personal electronic devices 101; one of which may serve as a central controller. The cloud 102 provides the hardware infrastructure (e.g. servers, databases, communication hardware, etc.) necessary to establish communication links to the probes and personal electronic devices, receive data from the probes including real-time actual soil sample analysis results and other information (e.g. pressure and temperature measurement data), store collected present and historical data transmitted by the probes in its database which can be accessed by the personal electronic devices. The probes and pedestals may communicate with the cloud 102 via cellular or satellite communication protocols, and/or local wireless/wired communications networks. Accordingly, the personal electronic devices 101 may be used to configuration probe controllers 60 of each probe 20 and control operation of the probes from a remote location near or far removed from the agriculture field where the probes are deployed.

Using all of the soil analysis data generated by the probes 20 (e.g. nutrient levels, etc.) and transmitted to the cloud system, the soil analysis software running on the cloud system may be configured to provide the soil water sample analysis results to the user's personal electronic device in various formats. For example, a color map of the agriculture field may be generated and presented to users on their personal electronic device display screens showing the concentration or level of the analyte of interest (e.g. nutrient levels like nitrate or other) in various regions of the agriculture field in different colors. Alternatively, the sample analysis results for the entire field may be presented in a tabular format or a combination of a color map and tabular data.

Each of probe controllers 60 may be operably and communicably coupled or linked to a central controller over a wireless local area network. In such a networked array of probes 20, a user's personal electronic device (i.e. cell phone, laptop, notebook, desktop, etc.) may be configured via executing a computer application or software to interact and control operation of the probes.

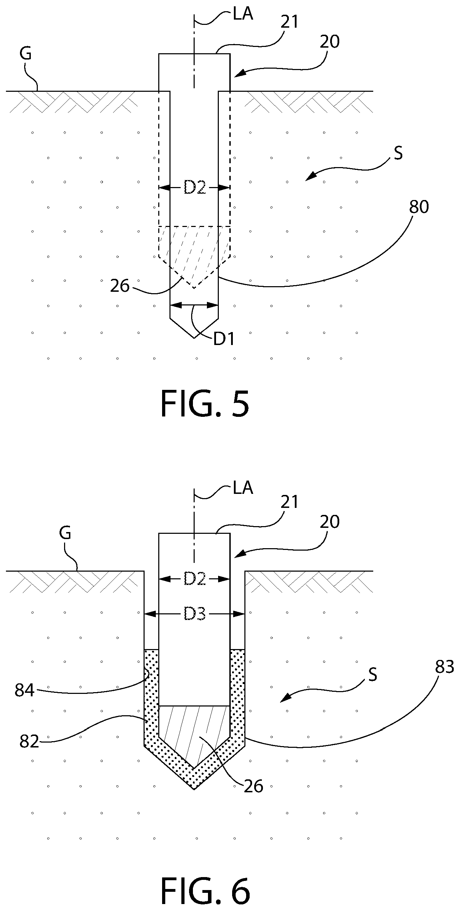

Returning now to the probes, intimate and preferably conformal contact between at least the porous media 26 of the probe 20 and surrounding soil S from which water is to be extracted for analysis is desirable to optimize drawing water into the probe. The probe 20 may be placed, driven or otherwise inserted directly into virgin soil such that the probe creates its own path and opening through the soil. This ensures intimate contact with soil. As shown in FIG. 5, where this is not possible or desirable either due to the density/composition of the soil and/or to protect the probe from damage during implantation, a pilot hole 80 may first be created (e.g. drilled, open via a spike or pry bar, etc.) in the soil having a diameter D1 slightly smaller than the maximum outer diameter D2 of the probe. When the probe is next vertically inserted into the pilot hole, intimate and conformal contact will be created between the probe and soil.

An alternative approach to creating intimate contact between the probe 20 and soil S is shown in FIG. 6. An insertion hole 83 is formed in the soil having a diameter D3 larger than the diameter D2 of the probe. This creates an annular gap or void 84 between the probe and sides of the hole 83. This void is then filled with a hydrophilic water-absorbing media 82 (aka slush powder) having a high matric potential. Examples of suitable materials which are commercially-available include silica flour, Bentonite, super absorbent polymers (SAP) (i.e. hydrogels), or other water-absorbing materials capable of producing a capillary or wicking action conducive to draw and capture the water from the hole surrounding the probe 20. SAP (hydrogels) will form gel when exposed to water creating a highly absorbent media capable of absorbing many times it weight with water. The hydrogels absorb water through forming hydrogen bonds with the water molecules. In operation, water is drawn from the soil into the water-absorbing media which is in intimate contract with both the exposed porous media of the probe and the sides of the hole 83, and then in turn is drawn through the porous media 26 into the probe for processing and analysis.

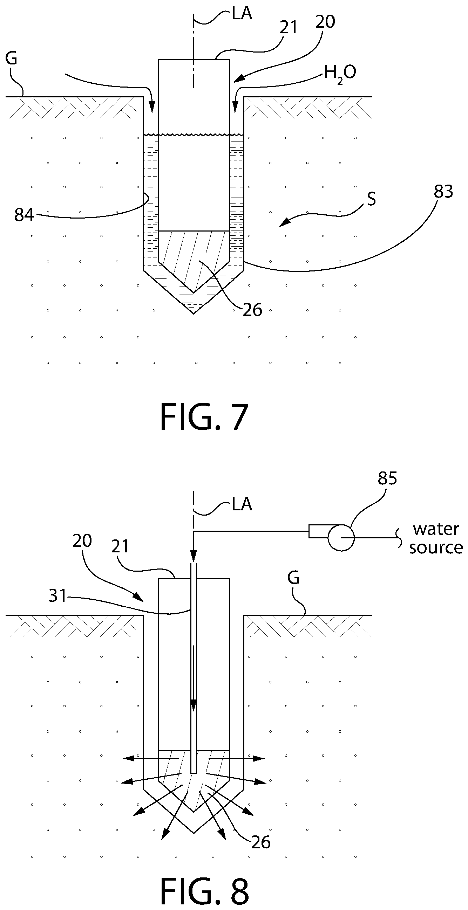

The annular void 84 may instead be filled directly with water from an available source preferably upon initial installation and insertion of the probe 20 in the soil S. This is shown in FIG. 7 and creates a native soil slurry surrounding the probe. In FIG. 8, water from a suitable in situ water source (e.g. tank, container, etc.) may be injected and back flowed through the probe and porous media 26 to mix with the soil surrounding the probe hole 83, thereby also forming a native soil slurry around the probe. A pressurizing water pump 85 or other source of water pressure may be provided to back flow water through the probe for injection into the annular void 84. A separate water injection flow conduit 31 may be provided. It bears noting that addition or injection of water into the hole or soil surrounding the probe may also be useful for characterizing the nutrient or other properties of the soil in instances where draught-like conditions may occur resulting in insufficient amounts of natural soil pore moisture/water to allow extraction through the probe for analysis.

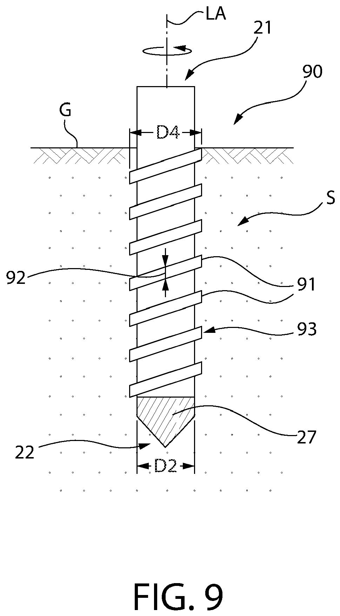

FIG. 9 shows a screw type sample probe 90 having an externally threaded body. Probe 90 includes a raised helical thread 91 which extends around the outer circumference of the probe body. Thread 91 has a larger outer diameter D4 than the diameter D2 of the cylindrical base portions of the probe. Helical thread 91 extends axially along the probe longitudinal axis LA between the top and bottom ends 21, 22 of the probe body as shown. Only the lower portions of the probe which will be implanted in the soil may contain the thread. Thread 91 may have raised axially elongated flat lands 93 between the valleys rather than sharply pointed threads to increase surface contact with and retention in the soil. The thread may terminate above the lower porous media tip 25 of the probe.

In lieu of linearly pushing, dropping, or otherwise inserting the threaded probe 90 into the soil, this probe may instead be rotated thereby axially/linearly translating the probe to the desired sampling depth. Threaded probe 90 advantageously eliminates the need to form a pilot or insertion hole first in the soil, and allows for easy extraction of the probe from the soil after useful service life is achieved or when terminating soil sampling. In addition, the threaded design creates greater ground engaging surface area which enhances intimate contact between the probe and soil for better induction of water into the probe. The probes stepped side profile further advantageously reduces the potential for vertical inflow of surface water in the hole surrounding the probe due to the circuitous path such surface water must take to travel downwards along the outside of the probe, thereby creating a greater resistance to flow than a straight linear path. Alternatively, in some installation, a smaller diameter pilot hole than the probe body may optionally be formed first in the soil similar to that described with respect to FIG. 5 above if desired to facilitate implantation of the threaded probe 90. This might be particularly useful for certain heavy soil types (e.g. clay).

### Hydrophilic Type Sampling Systems

Outside the scope of the present invention, a water-absorbing hydrophilic material capable of absorbing water may be used in the water sampling probe in lieu of a vacuum to extract moisture from the soil for nutrient or other soil parameter analysis. The hydrophilic material may be a hydrogel such as a superabsorbent polymer (SAP) also referred to as a "slush powder" in the art. The SAP acts as a wick which draws and moisture from the surrounding soil to the sampling probe via capillary type wicking action. The SAP may be mounted to the implanted sample probe and exposed in either direct contact with the soil, or indirectly in fluid communication with pore water in the soil via an intermediary hydrophilic fill media 82 (e.g. Bentonite, etc.) surrounding the probe in its hole such as shown in FIG. 6 and previously described herein.

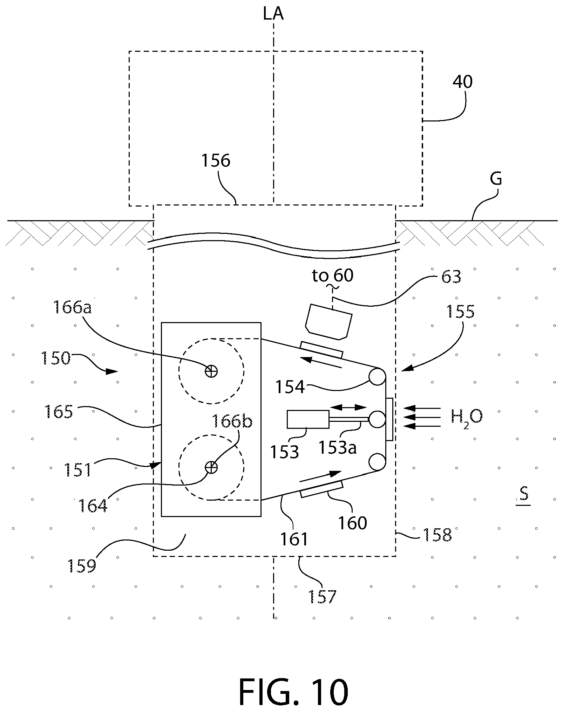

FIGS. 10-14 show a hydrophilic soil water sampling system using an SAP. Referring initially to FIG. 10, the system includes water sampling probe 150 which may be similar in configuration and construction of materials to probe 20 previously described herein but without the porous medium 26. Probe 150 has a tubular body including a top end 156, bottom end 157, sidewall 158 extending between the ends, and an internal cavity 159. The tubular body may have any suitable configuration or profile such as cylindrical, rectangular cuboid, etc. with a corresponding transverse cross section.

To perform in situ chemical analysis of the soil water sample remotely at each probe 150, the SAP material may be pre-combined with one or more reagents selected to cause a detectable color change in the presence of the analyte (e.g. nutrient) of interest in the collected water, which can then be analyzed at the collection site. The SAP and reagent(s) may be compressed and formed into absorbent cakes or blocks of material operable to absorb water when exposed to the soil surrounding the probe. Both the reagents and SAP may be in powdered form prior to being compressively formed under pressure into a block form.

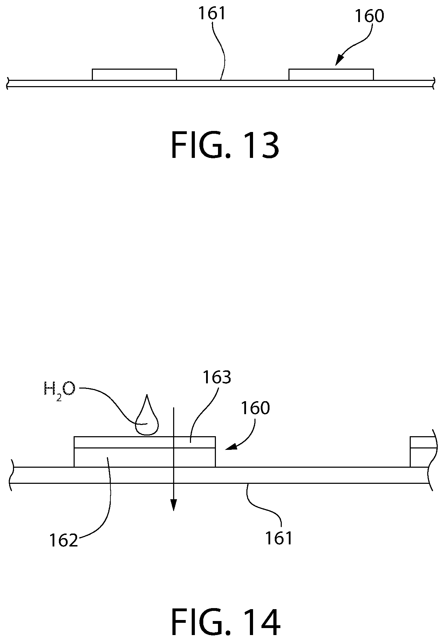

The compressed SAP may be in the form of tests pads 160 each having a generally rigid or semi-rigid block-like structure with a composite composition formed by a combination of the hydrogel or SAP and one or more reagents. Test pads 160 each include at least a first layer 162 comprising SAP and a first reagent. When requiring more than one reagent for a detectable color change of the water in the presence of the analyte, a second SAP and reagent layer 163 or more may be added. For example, the first reagent may be sulfanilic acid and the second reagent may be α-naphthylamine for nitrate detection and measurement. The first and second layers 162, 163 may be stacked and affixed to each other by any suitable means, including being compressed together in a press to form a single multi-layer composite block or test pad 160 (best shown in FIG. 14).

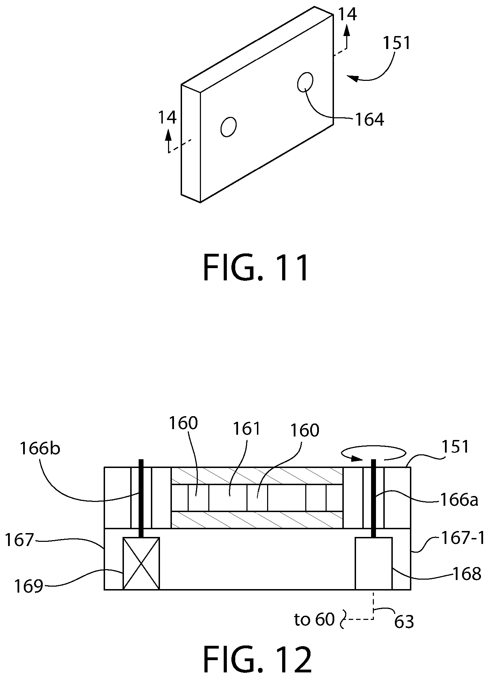

Because the probes 150 are intended to remain implanted and operable throughout of the entire growing, the probes preferably should be capable of performing multiple sample collection and analyses with minimal manual intervention and attention. The probe 150 may include transport mechanism configured to expose a plurality of new SAP test pads to the soil and its pore water each time a sample run is performed. The transport mechanism may be a replaceable and detachable sampling cassette 151 configured for securement inside the cavity 159 of the probe 150 body (best shown in FIG. 12). With additional reference to FIGS. 11-14, cassette 151 may be similar in construction and operation to a music/video tape cassette or micro-cassette. Cassette 151 includes a rigid rectangular cuboid case 165 defining an interior space, pair of reels 164, and a movable flexible substrate wound around the reels inside the case. Other configurations of cassette cases may of course be used. The substrate may be a preferably hydrophobic polymeric sampling tape 161 as one non-limiting example; however, other flexible materials may be used. The SAP test pads 160 are rigidly affixed and coupled to the tape 161 at spaced apart increments. Test pads 160 have a thickness (measured perpendicularly to the tape) which preferably is as low in profile as possible but sufficient to draw and hold sufficient water to react properly with the reagent(s).

An electric powered tape drive 167 is provided for operating the cassette 151 and advancing the test pads 160 in sequence for sampling. Referring particularly to FIGS. 10 and 12, tape drive 167 includes a pair of spaced apart spindles including an idler spindle 166b supported by a bearing 169 inside the drive housing 167-1 and a drive spindle 166a. Drive spindle 166a is coupled to an electric drive motor 168 of tape drive 167 which is operable to rotate the spindle for advancing the sampling tape 161 and test pads 160. Motor 168 may be located inside the drive housing 167-1 as shown. Motor 168 is operably and communicably coupled or linked to probe controller 60 via communication link 63. The probe controller 60 may thus be configured using appropriate configured software or instructions to control operation of the sampling cassette 151 including advancement of the tape and test pads for sampling and analysis of the reacted test pads after exposure to water containing the analyte (e.g. plant nutrient) of interest.

When deployed as shown in FIG. 10, a portion or loop of the substrate tape 161 may be pulled from the sampling cassette 151. The loop is guided around a pair or more of freely rotating guide spindles 154 affixed inside the internal cavity 159 of hydrophilic sampling probe 150. The guide spindles 154 are positioned to locate and expose the tape 161 and test pads 160 thereon to the soil through an open window and preferably screened sample collection window 155. The open/porous screening prevents the soil for ingress inside the probe 150 through the window. The substrate tape 161 is located proximate to window 155 so that a series of test pads 160 may be cycled to and past the window for intimate contact with the soil to absorb moisture.

To ensure intimate contact between the test pads 160 and soil through the screened window 155, probe 150 may further include a piston 153 which includes a linearly movable plunger 153a which acts on the inside facing surface of the movable tape 161 opposite the window 155. The piston 153 is operable to move the plunger 153a to engage, push, and displace the tape 161 outwards against the screen window. This places the test pad 160 on the outside facing opposite surface of the tape 161 into intimate contact with the surrounding soil to extract water therefrom (see, e.g. FIG. 10). Piston assembly 153 may be electric motor driven.

Probe 150 further includes a sample analysis device 152 which may be similar to device 36 previously described herein. When utilizing a color change in the test pads 160 to detect the presence and concentration of the analyte, sample analysis device 150 may be a colorimeter. Analysis device 150 is preferably located proximate and adjacent to tape 161 in a position which can detect and measure the analyte is test pad 160 after it has been exposed to soil water and changed color. This may be in the return side of the tape loop as shown in FIG. 12 rather than the feed side of the loop.

Operation of the probe 150 in a process or method for capturing and analyzing a soil water sample will now be briefly described with general reference to FIGS. 10-14. The process starts with the tape 161 loop already positioned as shown in FIG. 10. However, the plunger 153a may be in a first inward retracted position either completely disengaged from or lightly engaging the tape 161. The programmable probe controller 60 advances the sampling tape 161 in a first direction via operating the drive motor 168. Tape 161 is rotated to position an unused test pad 160 adjacent to screened sampling window 155. Piston 153 is operated by the probe controller 60 to move plunger 153a outwards to an extended position. The distal end of plunger 153a abuttingly engages the sampling tape 161 forcing it and the test pad 160 outwards into intimate contact with the soil through the screened sampling window 155. The test pad 160 remains in this intimate contact position until a sufficient amount of soil water is absorbed by the SAP-reagent composition of the pad for testing. Probe controller 60 may activate a timer for a predetermined period of time sufficient to ensure saturation of the test pad with water from the soil. Once the timer has expired, or other means used to ensure saturation of the test pad with a water sample, plunger 153a may be retracted. The sampling tape 161 is then advanced to position the saturated test pad 160 adjacent to the colorimeter (sample analysis device 152). The color change in the test pad is analyzed by the colorimeter to measure the concentration of the analyte. The colorimeter communicates the measurement to probe controller 60 via communication link 63 (wired or wireless).

The test pads 160 may be spaced apart on sampling tape 161 in intervals selected such that a new test pad is positioned adjacent to (but spaced apart from probe screened sampling window 155) and ready for the next test each time the tape is advanced to place a saturated test pad adjacent to the sample analysis device 152. The piston plunger 153a may remain retracted to prevent the new pad from being saturated until the next planned soil water extraction sampling cycle. The sampling may be performed on demand and/or at regular intervals (e.g. daily, weekly, etc.) according to a sampling schedule preprogrammed into probe controller 60. When the sampling cycle is initiated by controller 60, the piston plunger is again moved to the extended position for extracting a water sample in the same manner previously described above.

A thin protective film 161-1 may be provided on sampling tape 161 which is releasably adhered to and emplaced over the test pad 160 material. This overlay film protects the test pad or strip until needed. FIG. 17 shows one arrangement of a powered tape drive 167 for sampling cassette 151 which is configured for use with protection film 161-1. The protective film 161-1 is automatically peeled off of the substrate tape 161 and test pads 160 by a film guide spindle 161-3 to expose the test pads just before the pads are exposed to the soil water sample which is tested. The film 161-1 is then taken up and wound on a separate used film collection wheel 161-2, while the substrate tape 161 continues along its tape path as previously described herein (see directional film path arrow and substrate tape 161 path arrows). This film 161-1 protects the sensitive testing material from oxygen and moisture while the cassette in installed in the probe beneath the soil surface.

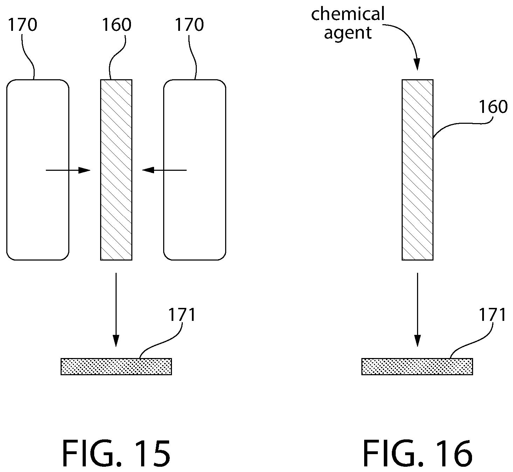

In an alternative sampling cassette 151, pure SAP test pads 160 without reagents may be used with sampling cassette 115 in the same manner described above for simply extracting the water sample from the soil. The captured water may then be liberated from the saturated test pad for analysis of the analyte by testing means separate from the SAP (e.g. colorimeter, test strip, etc.). Such means for liberating and extracting the water captured by the hydrophilic test pads 160 may include, for example without limitation, mechanical and chemical methods. FIG. 15 shows one non-limiting example of a mechanical method includes applying mechanical pressure or force on the saturated SAP test pad 160 to release the water such as via compressing the pad between a pair of mechanical compression members 170. The captured water is released to a chemical analysis device 171 such as a test strip for analysis of the analyte. Alternatively, the release water may be collected in a container such as collection chamber 30 for further processing and analysis in the system of FIG. 1 or another. FIG. 16 shows one non-limiting example of a chemical method may be changing the pH of the saturated SAP test pad 160 by adding a chemical agent (e.g. acid or other) to break the hydrogen bonds formed between the SAP material and water molecules, thereby releasing the water to the test strip or collection container for further processing and analysis. Other approaches may be used. It bears noting that these water liberation methods and pure SAP test pads may be used without a sampling cassette 151 in some alternative sampling probes.

While the foregoing description and drawings represent some example systems, it will be understood that various additions, modifications and substitutions may be made therein without departing from the scope of the invention, which is defined by the appended claims.

## Claims

1. A system (190) for collecting and analyzing soil water samples comprising:
a sample probe (20) comprising a filter media (26) arranged to contact the soil when embedded therein, the sample probe (20) configured for collecting a water sample from the soil; and
a sample processing sub-system (180) located proximately to the sample probe (20), the processing system operably coupled to the sample probe (20) and configured to extract and analyze the water sample for at least one analyte,
wherein the sample processing sub-system (180) includes:
a processor-based probe controller (60), the probe controller (60) configured to direct operation of the sub-system (180);
a vacuum device (32) configured for generating a vacuum in the sample probe (20) to extract a water sample from the soil;
a water sample analysis device (36) configured for measuring a concentration of an analyte in the water sample;
**characterized by**
a tape (161) for collecting the sample, the tape (161) movably disposed in a cassette (151), the cassette (151) disposed in an internal cavity (159) of the sample probe (20), the tape (161) further comprising a test pad (160) which collects the sample; and
a tape drive mechanism (167) operable to dispense the tape,
wherein the tape (161) comprises an active testing portion that lies external to the cassette (151) and is positioned for exposure to the soil through a window (155) of the sample probe (150), the active testing portion of the tape (161) movably guided around a pair of guide spindles (154) arranged inside the sampling probe when the tape drive mechanism (167) is operated.

2. The system (190) according to claim 1, wherein the probe controller (60) is configured to communicate with a remote electronic device (101) defining a central controller.

3. The system (190) according to claim 1, wherein the water sample analysis device (36) is selected from the group consisting of a colorimeter, ion selective electrodes, and ion exchange resins.

4. The system (190) according to claim 1, further comprising a removable film (161-1) covering at least the test pad (160).

5. The system (190) according to claim 1, wherein the tape (161) is wound around an idler spindle (166b) and a drive spindle (166a) operably coupled to the tape drive mechanism (167), the tape drive mechanism (167) operable to feed a continuous length of the tape (161) from the drive spindle (166a) to the idler spindle (166b).

6. The system (190) according to any of claims 1 to 5, further comprising a piston (153) configured to move the tape (161) into a window (155) in the sample probe (150).

7. The system (190) according to any of claims 1 to 6, further comprising a colorimeter (152) disposed to analyze the water sample on the tape (161).

8. The system (190) according to any preceding claim, further comprising:
a water system comprising a tank (351) containing sampling water located proximate to the sample probe (350), the water system configured to wet the soil surrounding the probe (350).

9. The system (190) according to claim 8, wherein the water system comprises a dispensing tube (354) fluidly coupled to the tank (351), the dispensing tube (354) configured to dispense the sampling water in a vicinity of the soil adjacent to the filter media (26).

10. The system (190) according to claim 9, wherein the dispensing tube (354) is arranged external to the probe (350).

11. The system (190) according to claim 9, wherein the dispensing tube (354) is arranged inside the probe (350).

## Patentansprüche

1. System (190) zum Sammeln und Analysieren von Bodenwasserproben mit:
einer Probeneinrichtung (20) mit einem Filtermedium (26), welches geeignet angeordnet ist für eine Kontaktierung des Bodens, bei Einbettung in diesem, wobei die Probeneinrichtung (20) zum Sammeln einer Wasserprobe aus dem Boden konfiguriert ist; und
einem Probenverarbeitungs-Subsystem (180), welches benachbart der Probeneinrichtung (20) angeordnet ist, wobei das Verarbeitungssystem betrieblich mit der Probeneinrichtung (20) gekoppelt ist und konfiguriert ist für ein Extrahieren und Analysieren mindestens eines Analyts der Wasserprobe,
wobei das Probenverarbeitungs-Subsystem (180) aufweist:
einen prozessor-basierten Proben-Controller (60), wobei der Proben-Controller (60) für eine Steuerung des Betriebs des Subsystems (180) konfiguriert ist;
eine Vakuum-Einrichtung (32), die für eine Erzeugung eines Vakuums in der Probeneinrichtung (20) zum Extrahieren einer Wasserprobe aus dem Boden konfiguriert ist;
eine Wasserproben-Analyseeinrichtung (36), die konfiguriert ist für das Messen einer Konzentration eines Analyten in der Wasserprobe,
**gekennzeichnet durch**
ein Band (161) zum Sammeln der Probe, wobei das Band (161) bewegbar in einer Kassette (151) angeordnet ist, die Kassette (151) in einer inneren Vertiefung (159) der Probeneinrichtung (20) angeordnet ist und das Band (161) des Weiteren ein Test-Pad (160) aufweist, welches die Probe sammelt; und
einen Band-Antriebsmechanismus (167), der betreibbar ist zur Abgabe des Bandes,
wobei das Band (161) einen aktiven Testbereich aufweist, der außerhalb der Kassette (151) liegt oder angeordnet ist und geeignet angeordnet ist für eine Aussetzung mit dem Boden durch ein Fenster (155) der Probeneinrichtung (150), wobei der aktive Testbereich des Bandes (161) bewegbar um ein Paar von Führungsspindeln (154) geführt ist, die innerhalb der Probeneinrichtung angeordnet sind, wenn der Band-Antriebsmechanismus (167) betrieben wird.

2. System (190) nach Anspruch 1, wobei der Proben-Controller (60) konfiguriert ist für eine Kommunikation mit einer beabstandet oder entfernt angeordneten elektronischen Einrichtung (101), die einen zentralen Controller bildet.

3. System (190) nach Anspruch 1, wobei die Wasserproben-Analyseeinrichtung (36) ein Kolorimeter ist und/oder von Ionen-selektiven Elektroden und/oder von Ionen-Austauschharzen gebildet ist.

4. System (190) nach Anspruch 1, des Weiteren mit einem entfernbaren Film (161-1), der mindestens das Test-Pad (160) abdeckt.

5. System (190) nach Anspruch 1, wobei das Band (161) um eine Leerlaufspindel (166b) und eine Antriebsspindel (166a), die betrieblich mit dem Band-Antriebsmechanismus (167) gekoppelt ist, gewunden ist, wobei der Band-Antriebsmechanismus (167) betreibbar ist zur Zuführung eines durchgehenden Längsabschnitts des Bandes (161) von der Antriebsspindel (166a) zu der Leerlaufspindel (166b).

6. System (190) nach einem der Ansprüche 1 bis 5, des Weiteren mit einem Kolben (153), der geeignet konfiguriert ist, um das Band (161) in ein Fenster (155) in der Probeneinrichtung (150) zu bewegen.

7. System (190) nach einem der Ansprüche 1 bis 6, des Weiteren mit einem Kolorimeter (152), welches geeignet angeordnet ist zur Analyse der Wasserprobe auf dem Band (161).

8. System (190) nach einem der vorhergehenden Ansprüche, des Weiteren mit
einem Wassersystem mit einem Tank (351), der Probenwasser beinhaltet und benachbart der Probeneinrichtung (350) angeordnet ist, wobei das Wassersystem geeignet konfiguriert ist, um den Boden in der Umgebung der Probe (350) zu befeuchten.

9. System (190) nach Anspruch 8, wobei das Wassersystem ein Abgaberohr oder einen Abgabeschlauch (354) aufweist, der fluidisch mit dem Tank (351) gekoppelt ist, wobei das Abgaberohr oder der Abgabeschlauch (354) konfiguriert ist für eine Abgabe des Probenwassers in der Nähe des Bodens benachbart dem Filtermedium (26).

10. System (190) nach Anspruch 9, wobei das Abgaberohr oder der Abgabeschlauch (354) außerhalb der Probe oder Probeneinrichtung (350) angeordnet ist.

11. System (190) nach Anspruch 9, wobei das Abgaberohr oder der Abgabeschlauch (354) innerhalb der Probe oder der Probeneinrichtung (350) angeordnet ist.

## Revendications

1. Dispositif (190) destiné à collecter et à analyser des échantillons d'eau du sol comprenant :
une sonde d'échantillonnage (20) comprenant un support de filtrage (26) agencé de manière à entrer en contact avec le sol dans lequel elle est enfoncée, la sonde d'échantillonnage (20) étant configurée de manière à collecter un échantillon d'eau dans le sol ; et
un sous-ensemble de traitement d'échantillon (180) situé à proximité de la sonde d'échantillonnage (20), l'ensemble de traitement étant couplé de manière opérationnelle à la sonde d'échantillonnage (20) et configuré de manière à extraire et à analyser au moins un analyte de l'échantillon d'eau,
dans lequel le sous-ensemble de traitement d'échantillon (180) comporte :
un contrôleur de sonde basé sur un processeur (60), le contrôleur de sonde (60) étant configuré de manière à commander le fonctionnement du sous-ensemble (180) ;
un dispositif de mise sous vide (32) configuré de manière à produire un vide dans la sonde d'échantillonnage (20) afin d'extraire un échantillon d'eau dans le sol ;
un dispositif d'analyse d'échantillon d'eau (36) configuré de manière à mesurer une concentration d'un analyte dans l'échantillon d'eau ;
**caractérisé par**
une bande (161) destinée à collecter l'échantillon, la bande (161) étant disposée de manière à pouvoir se déplacer dans une cassette (151), la cassette (151) étant disposée dans une cavité interne (159) de la sonde d'échantillonnage (20), la bande (161) comprenant, en outre, une garniture de test (160) qui collecte l'échantillon ; et
un mécanisme d'entraînement de bande (167) servant à distribuer la bande,
dans lequel la bande (161) comprend une partie de test active qui est située à l'extérieur de la cassette (151) et est positionnée afin d'assurer l'exposition au sol à travers une fenêtre (155) de la sonde d'échantillonnage (150), la partie de test active de la bande (161) étant guidée de manière à se déplacer autour d'une paire de broches de guidage (154) agencées à l'intérieur de la sonde d'échantillonnage lorsque le mécanisme d'entraînement de bande (167) est commandé.

2. Dispositif (190) selon la revendication 1, dans lequel le contrôleur de sonde (60) est configuré de manière à communiquer avec un dispositif électronique distant (101) définissant une unité centrale de commande.

3. Dispositif (190) selon la revendication 1, dans lequel le dispositif d'analyse d'échantillon d'eau (36) est sélectionné à partir du groupe constitué par un colorimètre, des électrodes sélectives d'ions et des résines échangeuses d'ions.

4. Dispositif (190) selon la revendication 1, comprenant, en outre, un film amovible (161-1) recouvrant au moins la garniture de test (160).

5. Dispositif (190) selon la revendication 1, dans lequel la bande (161) est enroulée autour d'une broche libre (166b) et d'une broche d'entraînement (166a) couplées de manière opérationnelle au mécanisme d'entraînement de bande (167), le mécanisme d'entraînement de bande (167) pouvant être utilisé afin de délivrer une longueur continue de la bande (161) à partir de la broche d'entraînement (166a) vers la broche libre (166b).

6. Dispositif (190) selon l'une quelconque des revendications 1 à 5, comprenant, en outre, un piston (153) configuré de manière à déplacer la bande (161) dans une fenêtre (155) sur la sonde d'échantillonnage (150).

7. Dispositif (190) selon l'une quelconque des revendications 1 à 6, comprenant, en outre, un colorimètre (152) agencé de manière à analyser l'échantillon d'eau sur la bande (161).

8. Dispositif (190) selon l'une quelconque des revendications précédentes, comprenant, en outre, :
un dispositif hydraulique comprenant un réservoir (351) contenant l'eau échantillonnée situé à proximité de la sonde d'échantillonnage (350), le dispositif hydraulique étant configuré de manière à humidifier le sol entourant la sonde (350).

9. Dispositif (190) selon la revendication 8, dans lequel le dispositif hydraulique comprend un tube de distribution (354) couplé de manière fluidique au réservoir (351), le tube de distribution (354) étant configuré de manière à distribuer l'eau échantillonnée à proximité du sol de manière adjacente au support de filtrage (26).

10. Dispositif (190) selon la revendication 9, dans lequel le tube de distribution (354) est agencé à l'extérieur de la sonde (350).

11. Dispositif (190) selon la revendication 9, dans lequel le tube de distribution (354) est agencé à l'intérieur de la sonde (350).
